# FASCICULE DE BREVET EUROPEEN

(11) **EP 1 014 927 B1**
(45) Date de publication et mention de la délivrance du brevet: **25.06.2003**
(21) Numéro de dépôt: 98902042.5
(22) Date de dépôt: 08.01.1998
(51) Int. Cl.: A61K 7/48, A61K 7/32, A61K 35/78, A61K 31/12, C07C 49/20

(54) **UTILISATION DES SHOGAOLS ET DES GINGEROLS POUR LA PREPARATION DE COMPOSITIONS DEODORANTES**
VERWENDUNG VON SHOGAOLEN UND GINGEROLEN ZUR HERSTELLUNG VON DEODORANZIEN
USE OF SHOGAOLS AND GINGEROLS FOR PREPARING DEODORANT COMPOSITIONS

(30) Priorité: 09.01.1997 FR 9700142; 09.01.1997 FR 9700143
(43) Date de publication de la demande: 05.07.2000
(73) Titulaire: Societe D'Etudes et de Recherches en Pharmacognosie - S E R P, 75006 Paris (FR)
(72) Inventeur: JEAN, Daniel, F-63270 Vic-le-Comte (FR); CARIEL, Léon, F-75006 Paris (FR)
(74) Mandataire: Goulard, Sophie
(86) Numéro de dépôt international: FR9800025
(87) Numéro de publication internationale: WO98030201

(56) Documents cités:
- EP-A- 0 334 967
- EP-A- 0 750 908
- WO-A-93/23061
- PATENT ABSTRACTS OF JAPAN vol. 13, no. 137 (C-582) [3485] & JP 63 303908 A (TSUNEO ARANAMI)
- DATABASE WPI Week 9729 Derwent Publications Ltd., London, GB; AN 97-316564 XP002044592 & JP 09 124 677 A (EBA KASHOHIN KK)
- DATABASE WPI Week 8934 Derwent Publications Ltd., London, GB; AN 89-246400 XP002044593 & JP 01 180 817 A (MORISHITA JINTAN KK)
- STN, Serveur de Bases de Données, XP002044589
- PATENT ABSTRACTS OF JAPAN vol. 95, no. 010 (C & JP 07 171209 A (MATSUSHITA ELECTRIC WORKS)
- STN, Serveur de Bases de Données, XP002044590
- STN, Serveur de Bases de Données, XP002044591
- DATABASE WPI Week 9439 Derwent Publications Ltd., London, GB; AN 94-313611 XP002066395 & JP 06 239 736 A (POLA CHEM IND)
- DATABASE WPI Week 9431 Derwent Publications Ltd., London, GB; AN 94-252699 XP002066396 & JP 06 183 959 A (POLA CHEM IND)
- DATABASE WPI Week 8217 Derwent Publications Ltd., London, GB; AN 82-33769E XP002066397 & JP 57 046 914 A (TSUMURA JUNTENDO KK)

## Description

La présente Invention se rapporte à l'utilisation des shogaols et des gingérols pour la préparation de compositions déodorantes.

L'odeur de la peau est produite par les sécrétions des glandes sudoripares et des glandes sébacées.

Les glandes sudoripares produisent la sueur qui est constituée par de l'eau plus ou moins chargée en sels minéraux et en substances organiques (acide lactique, urée, ...) tandis que les glandes sébacées sécrètent le sébum qui est principalement formé de corps gras : cholestérol et ses esters, acide stéarique, acide palmitique et leurs esters.

Des études [SHELLEY et al., *Arch. Dermatol. Syphilol*., 68, 430 (1973)] ont montré que les sécrétions sudoripares et sébacées sont inodores au moment de leur production. Les odeurs sont produites ultérieurement par l'action des bactéries commensales de la peau, action qui s'exerce essentiellement sur la sueur apocrine et le sébum en raison de leur richesse en substances organiques. La sueur écrine, qui est plus abondante mais plus pauvre en matières organiques que la sueur apocrine, n'interviendrait que faiblement dans la création des odeurs, encore qu'elle puisse jouer indirectement un rôle en favorisant par son volume la dispersion des composants de la sueur apocrine.

Ainsi, la décomposition des sécrétions des glandes sudoripares et des glandes sébacées par la flore bactérienne naturellement présente sur la peau, probablement en association avec une décomposition des protéines de la couche cornée, donne naissance à de nombreuses molécules fortement odorantes.

Face à ce phénomène qui existe sur toute la surface du corps mais qui se manifeste de façon particulièrement prononcée dans les régions pileuses (aisselles, plis inguinaux, région pubienne, ...) et palmo-plantaires, trois types de produits sont actuellement proposés, utilisables exclusivement par voie externe :
- des produits dits antitranspirants qui visent à réduire la production de sueur,
- des produits qui visent à limiter la pullulation microbienne locale et, ainsi, la décomposition des sécrétions cutanées, et
- des produits qui visent à neutraliser, dès leur émission, les substances issues de cette décomposition et responsables des mauvaises odeurs.

Si, d'une manière générale, ces produits présentent une certaine efficacité, ils présentent l'inconvénient, de par leur formulation et/ou leur présentation, d'être adaptés et, partant, limités à une application sur une zone corporelle particulière : aisselles, région génitale ou pieds, alors qu'il serait hautement souhaitable de pouvoir disposer de produits à usage universel, permettant de traiter toutes les zones corporelles concernées.

Par ailleurs, certains sujets souffrent de véritables maladies de la sudation telles que l'éphydrose qui correspond à une émission anormalement élevée de sueur, ou la bromidrose qui se caractérise par la production d'une sueur particulièrement fétide, et qui sont extrêmement gênantes pour eux-mêmes et pour leur entourage. Pour ces sujets, l'absence actuelle de produits aptes à traiter leur affection par voie générale constitue un véritable handicap.

Enfin, outre qu'un produit d'hygiène corporelle, notamment lorsqu'il s'agit un déodorant, se doit d'être dénué de toxicité, la méfiance dont font preuve, de façon de plus en plus marquée, de nombreux consommateurs vis-à-vis des produits d'origine chimique, les poussent à choisir de préférence des produits d'origine naturelle.

Le problème se pose, par conséquent, de disposer de produits capables de prévenir efficacement l'émission d'odeurs corporelles et ce, aussi bien lorsqu'ils sont administrés par voie générale que lorsqu'ils sont utilisés par voie locale, qui puissent, dans ce dernier cas, être appliqués sur toutes les zones du corps, qui soient, de plus, dépourvus de toxicité et qui présentent, en outre, l'avantage d'être des produits d'origine naturelle.

Or, dans le cadre de leurs travaux, les Inventeurs ont trouvé que les shogaols et les gingérols, composés qui répondent à la formule générale (I) ci-après : 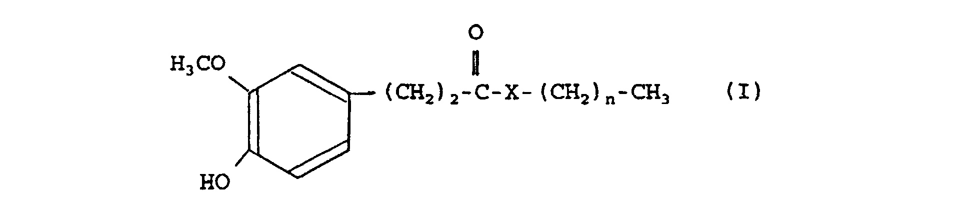 dans laquelle :
- X représente -CH=CH- dans le cas des shogaols, tandis que
- X représente -CH₂-CH(OH)- dans le cas des gingérols, et qui sont présents chez les plantes de la famille des Zingibéracées, sont, de manière inattendue, doués de propriétés déodorantes marquées, non seulement lorsqu'ils sont appliqués sur la peau mais également lorsqu'ils sont ingérés par voie orale, tout en étant atoxiques, ce qui leur confère un intérêt pour la préparation de compositions déodorantes pour le corps, utilisables par voie systémique ou par voie locale.

La présente Invention a, donc, pour objet l'utilisation d'au moins un composé choisi parmi le groupe qui comprend les shogaols et les gingérols pour la préparation d'une composition déodorante.

Conformément à l'Invention, le composé est de préférence choisi parmi les shogaols répondant à la formule générale (I) précédemment mentionnée dans laquelle, X représentant -CH=CH-, n est égal à 1, 2, 4, 6 ou 8, et qui sont dénommés respectivement [3]-shogaol, [4]-shogaol, [6]-shogaol, [8]-shogaol et [10]-shogaol, et les gingérols qui répondent à la formule générale (I) mentionnée précédemment dans laquelle, X représentant - CH₂-CH(OH)-, n est égal à 2, 4, 6 ou 8, et qui sont connus sous les noms respectifs de [4]-gingérol, [6]-gingérol, [8]-gingérol et [10]-gingérol.

Les shogaols et les gingérols peuvent être extraits, par tout procédé d'extraction en soi connu, des rhizomes de nombreuses Zingibéracées et, plus particulièrement, de celles appartenant aux genres *Alpinia* et *Zingiber.* A titre d'exemples, les [3]-shogaol, [6]-shogaol et [8]-shogaol peuvent être extraits des plantes du genre *Alpinia* telles qu'*Alpinia galanga* ou *Alpinia officinarum*, tandis que les [4]-shogaol, [10]-shogaol et les gingérols peuvent être extraits des plantes du genre *Zingiber* comme *Zingiber officinalis, Zingiber cassumunar* et *Zingiber zerumbet*.

Les shogaols et les gingérols peuvent également être obtenus par synthèse chimique, par exemple selon les procédés décrits par BANNO et MUKAIYAMA, *Bull. Chem. Soc. Japon*, 49(5), 1453-1454 (1976).

Selon un premier mode de mise en oeuvre préféré de l'Invention, le composé est utilisé sous la forme d'un extrait brut ou purifié d'une plante appartenant à la famille des Zingibéracées, ledit extrait étant éventuellement associé à une ou plusieurs autres substances actives et/ou à un ou plusieurs excipients convenablement choisis.

Selon une disposition avantageuse de ce mode préféré de mise en oeuvre, l'extrait brut de la Zingibéracée contient une quantité de composé comprise en poids entre environ 1 et 5% du poids sec dudit extrait.

Conformément à l'Invention, un tel extrait brut est obtenu à partir de rhizomes, frais ou secs, de ladite plante :
* par macération d'un broyat de ces rhizomes à une température comprise entre 10 et 35°C, suivie d'une ou plusieurs extractions à reflux de ce broyat, ou en soumettant un broyat desdits rhizomes à une percolation à une température comprise entre 10 et 35°C, chacune de ces opérations (macération, extractions à reflux et percolation) étant réalisées au moyen d'un solvant organique ou d'un mélange de solvants organiques appropriés et, si désiré,
* par élimination du ou des solvants de l'extrait ainsi obtenu pour l'obtention d'un extrait sec.

La macération du broyat de rhizomes, préalablement à son extraction, a principalement pour effet d'améliorer la mise en contact des tissus végétaux et du ou des solvants d'extraction. Sa durée peut être comprise entre une douzaine d'heures et une semaine selon l'état de fraîcheur des rhizomes utilisés.

Pour les opérations de macération, d'extractions à reflux et de percolation du broyat, on utilise, de préférence, des solvants organiques miscibles à l'eau et présentant un point d'ébullition relativement bas de manière à pouvoir être aisément éliminés ultérieurement par une simple évaporation, tels que l'éthanol, le méthanol, l'acétone ou leurs mélanges avec de l'eau. Toutefois, dans la mesure où les shogaols et les gingérols sont solubles dans de nombreux solvants organiques, il est également possible d'utiliser d'autres solvants organiques tels que l'acétate d'éthyle, l'éther éthylique, le chloroforme ou le chlorure de méthylène.

Selon une autre disposition avantageuse de ce mode de mise en oeuvre préféré de l'Invention, l'extrait purifié de la Zingibéracée contient une quantité de composé au moins égale en poids à 75% du poids sec dudit extrait.

Avantageusement, un tel extrait purifié est obtenu :
* en soumettant un extrait brut tel que précédemment défini, après élimination éventuelle du ou des solvants qu'il contient et/ou sa reprise dans de l'eau, à une ou plusieurs extractions à contre-courant au moyen d'un solvant organique ou un mélange de solvants organiques non miscibles à l'eau et, si désiré,
* par élimination du ou des solvants de l'extrait ainsi obtenu pour l'obtention d'un extrait sec.

Le ou les solvants organiques non miscibles à l'eau utiles pour effectuer lesdites extractions à contre-courant sont notamment choisis parmi l'acétate d'éthyle, l'éther éthylique, le chloroforme, le chlorure de méthylène et leurs mélanges.

De manière particulièrement préférée, l'extrait, qu'il soit brut ou purifié, est un extrait d'une Zingibéracée choisie parmi le groupe qui comprend *Alpinia galanga, Alpinia officinarum, Zingiber officinalis, Zingiber cassumunar* et *Zingiber zerumbet.*

Selon un autre mode de mise en oeuvre préféré de l'Invention, la composition déodorante est formulée pour une administration par voie orale, par exemple, sous la forme d'une poudre, d'une solution ou d'une suspension buvable, d'un sirop, de comprimés ou de gélules.

Selon encore un autre mode de mise en oeuvre préféré de l'Invention, la composition déodorante est formulée pour une application par voie locale, par exemple, sous la forme d'une poudre, d'une solution, d'une suspension, d'un gel ou d'une crème dermiques.

Selon encore un autre mode de mise en oeuvre de l'Invention, la composition déodorante est une composition pharmaceutique qui comprend le composé en tant que principe actif, éventuellement associé à un ou plusieurs autres principes actifs, et au moins un excipient pharmaceutiquement acceptable. Une telle composition pharmaceutique, qui peut être administrée par voie systémique et notamment par voie orale, ou par voie locale, trouve application dans le traitement des dyshydroses et, plus particulièrement, celui de la bromidrose.

En variante, la composition déodorante est une composition cosmétique qui comprend le composé en tant que substance active, éventuellement associé à une ou plusieurs autres substances actives, et au moins un excipient convenablement choisi.

Selon encore une autre variante, la composition déodorante est une composition diététique qui comprend le composé en tant que substance active, éventuellement associé à une ou plusieurs autres substances actives, et au moins un excipient convenablement choisi.

De telles compositions cosmétiques et diététiques peuvent avantageusement être utilisées en tant que déodorants corporels, pour prévenir l'émission de mauvaises odeurs liées notamment à la transpiration.

L'Invention englobe, pour l'utilisation précitée, à la fois des shogaols connus en eux-mêmes tels que les [4]-shogaol, [6]-shogaol, [8]-shogaol et [10]-shogaol [CONNELL et SUTHERLAND, *Aust. J. Chem*., 22(5), 1033-1043 (1969)] mais dont les propriétés déodorantes n'étaient ni connues, ni suggérées jusqu'à présent, et un shogaol nouveau qui est le [3]-shogaol, correspondant à la formule (II) ci-après : 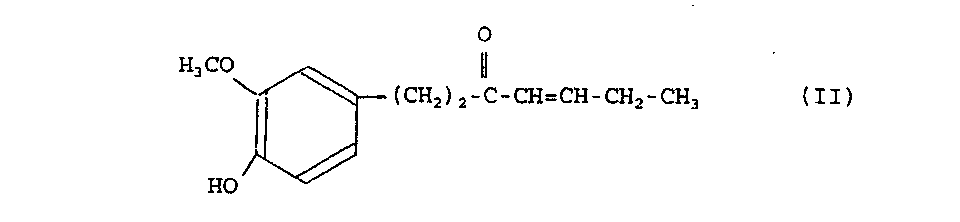 et qui représente, en raison de ses propriétés déodorantes particulièrement prononcées, le shogaol préférentiellement utilisé dans l'Invention.

Comme précédemment mentionné, le [3]-shogaol peut notamment être obtenu à partir d'une plante du genre *Alpinia,* par un procédé comprenant :
* la préparation d'un extrait brut à partir de rhizomes, frais ou secs, de cette plante,
* la purification de l'extrait brut ainsi obtenu, puis la séparation chromatographique de ce [3]-shogaol des autres shogaols susceptibles d'être présents dans l'extrait purifié.

L'Invention a, aussi, pour objet un extrait brut d'une plante appartenant au genre *Alpinia*, caractérisé en ce qu'il contient une quantité de [3]-shogaol comprise en poids entre environ 1 et 5% du poids sec dudit extrait.

Conformément à l'Invention, cet extrait est obtenu à partir de rhizomes, frais ou secs, de ladite plante :
* par macération d'un broyat de ces rhizomes à une température comprise entre 10 et 35°C, suivie d'une ou plusieurs extractions à reflux de ce broyat, ou en soumettant un broyat desdits rhizomes à une percolation à une température comprise entre 10 et 35°C, chacune de ces opérations (macération, extractions à reflux et percolation) étant réalisées au moyen d'un solvant organique ou d'un mélange de solvants organiques appropriés et, si désiré,
* par élimination du ou des solvants de l'extrait ainsi obtenu pour l'obtention d'un extrait sec.

Selon un mode de réalisation préféré de l'Invention, ledit extrait brut est un extrait d'*Alpinia galanga*.

L'Invention a, encore, pour objet un extrait purifié d'une plante appartenant au genre *Alpinia*, caractérisé en ce qu'il contient une quantité de [3]-shogaol au moins égale en poids à 75% du poids sec dudit extrait.

Avantageusement, cet extrait purifié est obtenu :
* en soumettant un extrait brut tel que précédemment défini, après élimination éventuelle du ou des solvants qu'il contient et/ou sa reprise dans de l'eau, à une ou plusieurs extractions à contre-courant au moyen d'un solvant organique ou un mélange de solvants organiques non miscibles à l'eau et, si désiré,
* par élimination du ou des solvants de l'extrait ainsi obtenu pour l'obtention d'un extrait sec.

De manière préférée, cet extrait purifié est un extrait *d'Alpinia galanga*.

De tels extraits bruts ou purifiés d'*Alpinia* se sont révélés être eux-mêmes doués de propriétés déodorantes prononcées lorsqu'ils sont administrés par voie générale ou appliqués par voie locale, tout en étant dénués de toxicité et conviennent, de ce fait, particulièrement bien à la préparation d'une composition déodorante conforme à l'Invention. A cette fin, ils sont susceptibles d'être utilisés soit tels quels sous la forme de poudres sèches ou de solutions aqueuses ou alcooliques, soit sous la forme de formulations plus complexes, éventuellement en association avec d'autres substances actives.

La présente Invention a, en outre, pour objet une composition déodorante, caractérisée en ce qu'elle comprend, en tant que substance active, du [3]-shogaol.

Selon un premier mode de réalisation, cette composition est une composition pharmaceutique qui comprend le[3]-shogaol en tant que principe actif, éventuellement associé à un ou plusieurs autres principes actifs, et au moins un excipient pharmaceutiquement acceptable.

Selon un autre mode de réalisation, cette composition est une composition cosmétique qui comprend le [3]-shogaol en tant que substance active, éventuellement associé à une ou plusieurs autres substances actives, et au moins un excipient convenablement choisi.

Selon encore un autre mode de réalisation, cette composition est une composition diététique qui comprend le[3]-shogaol en tant que substance active, éventuellement associé à une ou plusieurs autres substances actives, et au moins un excipient convenablement choisi.

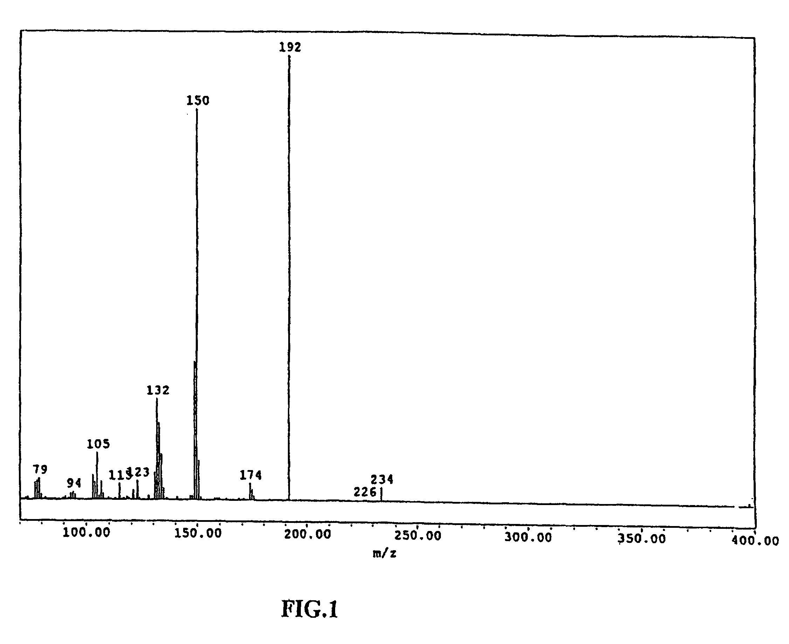

Outre les dispositions qui précèdent, l'Invention comprend encore d'autres dispositions qui ressortiront du complément de description qui suit, qui se réfère à des exemples de préparation d'extraits de rhizomes de plantes de la famille des Zingibéracées susceptibles d'être utilisés conformément à l'Invention, à un exemple d'obtention du [3]-shogaol et à des exemples de démonstration des activités déodorantes de ces extraits et de ce [3]-shogaol, ainsi qu'à la Figure 1 annexée qui représente le spectre de masse du [3]-shogaol.

Il va de soi, toutefois, que ces exemples sont donnés uniquement à titre d'illustrations de l'objet de l'Invention dont ils ne constituent en aucune manière une limitation.

### EXEMPLE 1 : PREPARATION D'UN EXTRAIT BRUT DE RHIZOMES D'ALPINIA GALANGA

Un kilo de rhizomes frais *d'Alpinia galanga* est broyé grossièrement en veillant à ne pas provoquer un échauffement trop important des parties broyées. On détermine la teneur en eau du broyat ainsi obtenu et on le met à macérer dans 7 litres d'éthanol dont le titre est choisi de manière à ce que, compte-tenu de la teneur en eau du broyat, le solvant de macération soit de l'éthanol à 50%.

Après 24 heures de macération à 20°C environ, le broyat est extrait à reflux par le solvant de macération pendant 30 minutes. Ce solvant est écarté et remplacé par un poids égal d'éthanol à 50%, et l'on extrait à nouveau à reflux le broyat pendant 30 minutes. L'opération est renouvelée une fois.

Les 3 extraits obtenus sont réunis (constituant ainsi un volume d'environ 19 litres), filtrés sur papier, puis évaporés à sec sous pression réduite.

On obtient un résidu qui pèse environ 50 g, soit un rendement approximatif de 30% par rapport au poids sec des rhizomes. Cet extrait renferme les différents shogaols présents dans les rhizomes d'*Alpinia galanga* ( [3]-shogaol, [6]-shogaol et [8]-shogaol) et sa teneur en [3]-shogaol est généralement comprise entre 1 et 5% (p/p) selon les rhizomes utilisés.

### EXEMPLE 2 : PREPARATION D'UN EXTRAIT PURIFIE DE RHIZOMES D'ALPINIA GALANGA

50 g d'un extrait brut, préparé conformément à l'exemple 1, sont repris dans 1 litre d'eau distillée et l'ensemble est porté à ébullition pendant 1 minute sous agitation constante. On poursuit l'agitation jusqu'à obtenir une complète homogénéisation de cet extrait et on le laisse refroidir. On le soumet alors à 4 extractions successives à contre-courant réalisées chacune avec 100 ml d'éther éthylique.

Les solutions éthérées sont réunies ; elles sont additionnées de sulfate de sodium anhydre pour éliminer l'eau qu'elles renferment ; elles sont filtrées sur papier et évaporées à sec sous pression réduite.

On obtient ainsi un résidu qui pèse 6,8 g, soit un rendement d'environ 4% par rapport au poids sec des rhizomes. Cet extrait, qui contient majoritairement du [3]-shogaol, a une teneur en [3]-shogaol qui est généralement supérieure à 75% (p/p).

### EXEMPLE 3 : OBTENTION DU [3]-SHOGAOL

Le [3]-shogaol peut être obtenu à partir de rhizomes d'*Alpinia galanga* en préparant un extrait brut de ces rhizomes conformément à l'exemple 1, en purifiant ensuite cet extrait conformément à l'exemple 2 puis, en soumettant l'extrait ainsi purifié à des élutions successives sur colonnes de gel de silice, par exemple de la manière suivante.

10 g d'un extrait purifié, préparé conformément à l'exemple 2, sont additionnés de 100 g d'un gel de silice G60 et de 500 ml de chloroforme sous agitation constante. Dès que ce mélange est homogène, il est évaporé à sec sous pression réduite de manière à obtenir une poudre.

On dépose cette poudre au sommet d'une colonne de 10 cm de diamètre et de 50 cm de hauteur, contenant également du gel de silice G60 dans de l'éther de pétrole. On élue d'abord par de l'éther de pétrole jusqu'à ce que le résidu soit inférieur à 0,1% (10 litres environ d'éther de pétrole sont nécessaires pour parvenir à ce stade), puis par 12 litres de benzène et, enfin, par 8 litres de chloroforme.

La phase chloroformique est évaporée à sec sous pression réduite laissant la place à un résidu d'environ 2,3 g. Ce résidu est alors soumis à une chromatographie préparative sur une colonne de 5 cm de diamètre et de 20 cm de hauteur, remplie de gel de silice C18, et en utilisant un mélange eau/acétonitrile (70/30) comme gradient d'élution. La fraction contenant le [3]-shogaol est éluée dans un temps compris entre 5 et 7 minutes pour un débit de 30 ml/mn.

Le [3]-shogaol peut être identifié par une chromatographie liquide à haute pression (HPLC) couplée à une spectrométrie de masse.

La Figure 1 montre le spectre de masse du [3] - shogaol obtenu avec un spectromètre Waters Integrity® à ionisation par impact électronique couplé à une HPLC réalisée sur colonne Nucléosil® (4,6 x 150 mm en 3 µm) avec une élution isocratique par un mélange isopropanol/cyclohexane (10/90).

### EXEMPLE 4 : PREPARATION D'UN EXTRAIT BRUT DE RHIZOMES DE ZINGIBER OFFICINALIS

En utilisant un protocole opératoire identique à celui décrit dans l'exemple 1, on obtient à partir d'un kilo de rhizomes frais de *Zingiber officinalis* un résidu qui pèse environ 52 g, soit un rendement approximatif de 35% par rapport au poids sec des rhizomes.

Cet extrait qui renferme les gingérols présents dans les rhizomes de *Zingiber officinalis* ([6] - gingérol, [8]-gingérol et [10]-gingérol), présente une teneur en gingérols qui est généralement comprise entre 1 et 5% (p/p) selon les rhizomes utilisés.

### EXEMPLE 5 : PREPARATION D'UN EXTRAIT PURIFIE DE RHIZOMES DE ZINGIBER OFFICINALIS

En soumettant 50 g d'un extrait brut préparé conformément à l'exemple 4 à une purification dans des conditions identiques à celles décrites dans l'exemple 2, on obtient un résidu qui pèse 8,2 g, soit un rendement d'environ 5,5% par rapport au poids sec des rhizomes. Cet extrait a une teneur en gingérols qui est généralement supérieure à 75% (p/p).

### EXEMPLE 6 : ACTIVITE DEODORANTE D'UN EXTRAIT BRUT DE RHIZOMES D'ALPINIA GALANGA

### a) Activité déodorante par voie systémique :

L'activité déodorante par voie systémique d'un extrait brut de rhizomes d'*Alpinia galanga*, préparé conformément à l'exemple 1, a été testée chez un groupe de 20 personnes en leur administrant par voie orale cet extrait préalablement formulé sous forme de gélules, et a été comparée à l'activité d'un placebo.

Pour ce faire, 1 kilo d'extrait brut a été mélangé intimement avec 1 kilo de maltodextrine dans un broyeur à couteaux afin d'assurer une meilleure homogénéité au mélange et d'obtenir une poudre mobile non collante. Cette poudre a ensuite été répartie dans des gélules n°0 de manière à obtenir un dosage unitaire de 250 mg d'extrait brut.

Ces gélules ont été administrées oralement à un premier groupe (groupe A) de 20 personnes comprenant 10 hommes et 10 femmes d'âge compris entre 17 et 62 ans, pendant 10 jours à raison de 3 gélules par jour en une seule prise, tandis qu'un groupe témoin (groupe T), composé également de 10 hommes et de 10 femmes et statistiquement comparable au précédent, a reçu, pendant 10 jours et en une seule prise orale, 3 gélules par jour contenant chacune 250 mg de lactose.

Les sujets des deux groupes se sont abstenus d'utiliser des déodorants à usage local pendant toute la semaine précédant le début du test, ainsi que pendant toute la durée de ce test.

L'efficacité de l'extrait brut conforme à l'Invention et celle du placebo ont été évaluées de façon sensorielle au niveau de la zone axillaire des sujets, conformément aux tests classiquement utilisés pour les déodorants, et ce, le premier jour du test (J0) avant la première prise, aux 3ème et 6ème jours (J3 et J6), au dernier jour du test (J10), ainsi qu'aux 3ème et 6ème jours suivant la fin de ce test (J13 et J16).

Les résultats obtenus, exprimés sous la forme de scores allant de 0 à 5 - la valeur 0 correspondant à une absence d'efficacité déodorante et la valeur 5 à une efficacité déodorante absolue (absence totale d'émission d'odeurs) -, sont présentés dans le Tableau 1 ci-après.

**TABLEAU 1**

| **JOURS** | **GROUPE A** | **GROUPE T** |
|---|---|---|
| J0 | 0 | 0 |
| J3 | 2 | 0 |
| J6 | 4 | 0 |
| J10 | 4 | 1 |
| J13 | 3 | 0 |
| J16 | 0 | 0 |

### b) Activité déodorante par voie locale :

L'activité déodorante par voie locale d'un extrait brut de rhizomes d'*Alpinia galanga*, préparé conformément à l'exemple 1, a également été testée chez un groupe de 20 personnes versus placebo.

Pour ce faire, un premier groupe (groupe A) comprenant 10 hommes et 10 femmes d'âge compris entre 17 et 62 ans s'est appliqué par pulvérisations, sur les deux zones axillaires, pendant 3 jours et à raison d'une seule pulvérisation par jour, 0,5 ml d'une solution d'éthanol à 50% comprenant 1% dudit extrait tandis qu'un deuxième groupe (groupe T), composé également de 10 hommes et de 10 femmes et statistiquement comparable au premier, s'est appliqué, dans les mêmes conditions, 0,5 ml d'éthanol à 50%.

Tous les sujets se sont abstenus d'utiliser des déodorants à usage local pendant toute la semaine précédant le début du test, ainsi que pendant toute la durée de ce test.

L'efficacité de l'extrait brut conforme à l'Invention et celle du placebo ont été évaluées de façon sensorielle au niveau de la zone axillaire des sujets et ce, le premier jour du test avant la première application (T0), puis 24 heures (T24), 48 heures (T48) et 72 heures (T72) après le début de ce test.

Les résultats, exprimés sous la forme de scores allant de 0 à 5 - la valeur 0 correspondant à une absence d'efficacité déodorante et la valeur 5 à une efficacité déodorante absolue - sont présentés dans le Tableau 2 ci-dessous.

**TABLEAU 2**

| **HEURES** | **GROUPE A** | **GROUPE T** |
|---|---|---|
| T0 | 0 | 0 |
| T24 | 2 | 0 |
| T48 | 3 | 0 |
| T72 | 3 | 0 |

### EXEMPLE 7 : ACTIVITE DEODORANTE DU [3]-SHOGAOL

### a) Activité déodorante par voie systémique :

L'activité déodorante par voie systémique du [3]-shogaol a été vérifiée chez un groupe de 20 personnes (groupe A) versus placebo (groupe T) par un test similaire à celui utilisé selon l'exemple 6 a) pour évaluer l'efficacité, par voie systémique, de l'extrait brut de rhizomes *d'Alpinia galanga*, à cette différence près que ces personnes ont reçu des gélules contenant 25 mg de [3]-shogaol au lieu de recevoir des gélules renfermant 250 mg dudit extrait.

Les résultats obtenus, également exprimés sous la forme de scores allant de 0 (absence d'efficacité) à 5 (efficacité absolue), sont présentés dans le Tableau 3 ci-après.

**TABLEAU 3**

| **JOURS** | **GROUPE A** | **GROUPE T** |
|---|---|---|
| J0 | 0 | 0 |
| J3 | 4 | 1 |
| J6 | 5 | 0 |
| J10 | 5 | 1 |
| J13 | 4 | 0 |
| J16 | 1 | 0 |

### b) Activité déodorante par voie locale :

### - préparation d'une solution cosmétique de [3]-shogaol, apte à être utilisée comme déodorant par voie locale :

10 g de [3]-shogaol sont dissous à froid dans 250 ml d'éthanol à 96%. Lorsque leur dissolution est complète, on ajoute 50 ml de monopropylène glycol et 200 ml d'eau purifiée. La solution obtenue est filtrée, puis conditionnée en pulvérisateurs.

### - évaluation de l'efficacité de la solution cosmétique de [3]-shogaol :

Elle a été réalisée chez un groupe de 20 personnes (groupe A) versus placebo (groupe T) par un test analogue à celui utilisé dans l'exemple 6 b) pour apprécier l'activité déodorante susceptible d'être présentée, par voie locale, par l'extrait brut d'*Alpinia galanga*.

Toutefois, dans la présente évaluation, les personnes du groupe A se sont appliquées, à chaque pulvérisation, 0,5 ml de solution cosmétique de [3]-shogaol tandis que les personnes du groupe T se sont appliquées 0,5 ml d'une solution éthanolique contenant 10% de monopropylène glycol et 40% d'eau.

Les résultats obtenus au cours de cette évaluation, également exprimés sous la forme de scores allant de 0 (absence d'efficacité) à 5 (efficacité absolue), sont présentés dans le Tableau 4 ci-dessous.

**TABLEAU 4**

| **HEURES** | **GROUPE A** | **GROUPE T** |
|---|---|---|
| T0 | 0 | 0 |
| T24 | 4 | 0 |
| T48 | 4 | 1 |
| T72 | 5 | 0 |

### EXEMPLE 8 : ACTIVITE DEODORANTE D'UN EXTRAIT BRUT DE RHIZOMES DE ZINGIBER OFFICINALIS

### a) Activité déodorante par voie systémique :

L'activité déodorante par voie systémique d'un extrait brut de rhizomes de *Zingiber officinalis*, préparé conformément à l'exemple 4, a été testée chez un groupe de 20 personnes (groupe A) versus placebo (groupe T) par un test analogue à celui utilisé dans l'exemple 6 a) pour évaluer l'efficacité, par voie systémique, de l'extrait brut de rhizomes d'*Alpinia galanga*.

Les résultats obtenus, également exprimés sous la forme de scores allant de 0 (absence d'efficacité) à 5 (efficacité absolue) sont présentés dans le Tableau 5 ci-après.

**TABLEAU 5**

| **JOURS** | **GROUPE A** | **GROUPE T** |
|---|---|---|
| J0 | 0 | 0 |
| J3 | 2 | 0 |
| J6 | 2 | 0 |
| J10 | 3 | 1 |
| J13 | 2 | 0 |
| J16 | 0 | 0 |

### b) Activité déodorante par voie locale :

L'activité déodorante par voie locale d'un extrait brut de *Zingiber officinalis*, préparé conformément à l'exemple 4, a également été testée chez un groupe de 20 personnes (groupe A) versus placebo (groupe T) par un test analogue à celui utilisé dans l'exempl 6 b) pour évaluer l'activité déodorante susceptible d'être présentée, par voie locale, par l'extrait brut d'*Alpinia galanga*.

Les résultats, également exprimés sous la forme de scores allant de 0 (absence d'efficacité) à 5 (efficacité absolue) sont présentés dans le Tableau 6 ci-dessous.

**TABLEAU 6**

| **HEURES** | **GROUPE A** | **GROUPE T** |
|---|---|---|
| T0 | 0 | 0 |
| T24 | 2 | 0 |
| T48 | 2 | 0 |
| T72 | 2 | 0 |

### EXEMPLE 9 : ACTIVITE DEODORANTE D'UN EXTRAIT PURIFIE DE RHIZOMES DE ZINGIBER OFFICINALIS

### b) Activité déodorante par voie systémique :

L'activité déodorante par voie systémique d'un extrait purifié de rhizomes de *Zingiber officinalis*, préparé conformément à l'exemple 5, a été vérifiée chez un groupe de 20 personnes (groupe A) versus placebo (groupe T) par un test similaire à celui utilisé selon l'exemple 6 a) pour évaluer l'efficacité, par voie systémique, de l'extrait brut de rhizomes d'*Alpinia galanga*, à cette différence près que ces personnes ont reçu des gélules contenant 25 mg d'extrait purifié de rhizomes de *Zingiber officinalis* au lieu de recevoir des gélules renfermant 250 mg d'extrait brut de rhizomes d'*Alpinia galanga*.

Les résultats obtenus, également exprimés sous la forme de scores allant de 0 (absence d'efficacité) à 5 (efficacité absolue) sont présentés dans le Tableau 7 ci-après.

**TABLEAU 7**

| **JOURS** | **GROUPE A** | **GROUPE T** |
|---|---|---|
| J0 | 0 | 0 |
| J3 | 3 | 1 |
| J6 | 3 | 0 |
| J10 | 4 | 1 |
| J13 | 2 | 0 |
| J16 | 1 | 0 |

### b) Activité déodorante par voie locale :

### - préparation d'une solution cosmétique à partir d'un extrait purifié de Zingiber officinalis, apte à être utilisée comme déodorant par voie locale :

10 g d'un extrait purifié, préparé conformément à l'exemple 5, sont dissous à froid dans 250 ml d'éthanol à 96%. Lorsque leur dissolution est complète, on ajoute 50 ml de monopropylène glycol et 200 ml d'eau purifiée. La solution obtenue est filtrée, puis conditionnée en pulvérisateurs.

### - évaluation de l'efficacité de ladite solution cosmétique :

Elle a été réalisée chez un groupe de 20 personnes (groupe A) versus placebo (groupe T) par un test analogue à celui utilisé selon l'exemple 6 b) pour apprécier l'activité déodorante susceptible d'être présentée, par voie locale, par l'extrait brut de rhizomes d'*Alpinia galanga*.

Toutefois, dans la présente évaluation, les personnes du groupe A se sont appliquées, à chaque pulvérisation, 0,5 ml de solution cosmétique, tandis que les personnes du groupe T se sont appliquées 0,5 ml d'une solution éthanolique contenant 10% de monopropylène glycol et 40% d'eau.

Les résultats obtenus au cours de cette évaluation, également exprimés en scores allant de 0 (absence d'efficacité) à 5 (efficacité absolue), sont rassemblés dans le Tableau 8 ci-dessous.

**TABLEAU 8**

| **HEURES** | **GROUPE A** | **GROUPE T** |
|---|---|---|
| T0 | 0 | 0 |
| T24 | 3 | 0 |
| T48 | 2 | 1 |
| T72 | 3 | 0 |

Ainsi que cela ressort de ce qui précède, l'Invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente Invention.

Ainsi, entre notamment dans le cadre de l'Invention l'utilisation de tout shogaol ou gingérol qui serait obtenu par voie de synthèse pour la préparation d'une composition déodorante ainsi que toute composition déodorante contenant un tel shogaol ou gingérol.

## Revendications

1. Utilisation d'au moins un composé choisi parmi le groupe constitué des shogaols et des gingérols pour la préparation d'une composition déodorante pour le corps destinée à une administration par voie orale.

2. Utilisation selon la revendication 1, **caractérisée en ce que** le composé répond à la formule générale (I) : 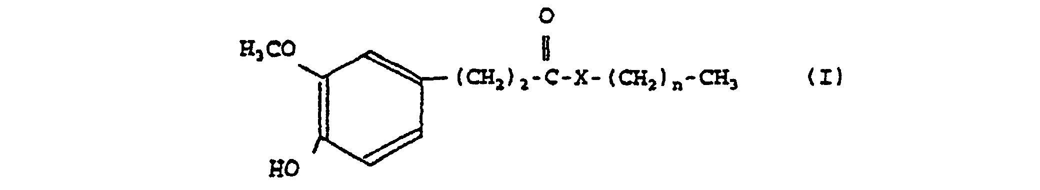 dans laquelle X représente :
- soit -CH=CH-, auquel cas n est égal à 1, 2, 4, 6 ou 8 ;
- soit -CH₂-CH(OH)-, auquel cas n est égal à 2, 4, 6 ou 8.

3. Utilisation selon la revendication 1 ou la revendication 2, **caractérisée en ce que** le composé est utilisé sous la forme d'un extrait brut ou purifié d'une plante appartenant à la famille des Zingibéracées, ledit extrait étant éventuellement associé à une ou plusieurs autres substances actives et/ou un ou plusieurs excipients convenablement choisis.

4. Utilisation selon la revendication 3, **caractérisée en ce que** ledit extrait brut contient une quantité de composé comprise en poids entre 1 et 5% du poids sec dudit extrait.

5. Utilisation selon la revendication 3 ou la revendication 4, **caractérisée en ce que** l'extrait brut de la plante appartenant à la famille des Zingibéracées est obtenu à partir de rhizomes, frais ou secs, de ladite plante :
* par macération, d'un broyat de ces rhizomes à une température comprise entre 10 et 35°C, suivie d'une ou plusieurs extractions à reflux de ce broyat, ou en soumettant un broyat desdits rhizomes à une percolation à une température comprise entre 10 et 35°C, chacune de ces opérations (macération, extractions à reflux et percolation) étant réalisées au moyen d'un solvant organique ou d'un mélange de solvants organiques appropriés et, si désiré,
* par élimination du ou des solvants de l'extrait ainsi obtenu pour l'obtention d'un extrait sec.

6. Utilisation selon la revendication 3, **caractérisée en ce que** l'extrait purifié contient une quantité de composé au moins égale en poids à 75% du poids sec dudit extrait.

7. Utilisation selon la revendication 3 ou la revendication 6, **caractérisée en ce que** l'extrait purifié de la plante appartenant à la famille des Zingibéracées est obtenu :
* en soumettant un extrait brut selon la revendication 4 ou la revendication 5, après élimination éventuelle du ou des solvants qu'il contient et/ou sa reprise dans de l'eau, à une ou plusieurs extractions à contre-courant au moyen d'un solvant organique ou un mélange de solvants organiques non miscibles à l'eau et, si désiré,
* par élimination du ou des solvants de l'extrait ainsi obtenu pour l'obtention d'un extrait sec.

8. Utilisation selon l'une quelconque des revendications 3 à 7, **caractérisée en ce que** la plante de la famille des Zingibéracées est choisie parmi le groupe qui comprend les espèces *Alpinia galanga, Alpinia officinarum, Zingiber officinalis, Zingiber cassumunar et Zingiber zerumbet.*

9. Utilisation selon l'une quelconque des revendications précédentes, **caractérisée en ce que** la composition déodorante est une composition pharmaceutique comprenant le composé en tant que principe actif, éventuellement associé à un ou plusieurs autres principes actifs, et au moins un excipient pharmaceutiquement acceptable.

10. Utilisation selon l'une quelconque des revendications 1 à 8, **caractérisée en ce que** la composition déodorante est une composition diététique comprenant le composé en tant que substance active, éventuellement associé à une ou plusieurs autres substances actives, et au moins un excipient convenablement choisi.

11. Shogaol répondant à la formule générale (I) dans laquelle X représente -CH=CH- et n est égal à 1.

12. Extrait brut d'une plante appartenant au genre *Alpinia*, **caractérisé en ce qu'**il contient une quantité de shogaol selon la revendication 11 comprise en poids entre environ 1 et 5% du poids sec dudit extrait.

13. Extrait brut selon la revendication 12, **caractérisé en ce qu'**il est obtenu à partir de rhizomes, frais ou secs, de ladite plante :
* par macération d'un broyat de ces rhizomes, à une température comprise entre 10 et 35°C, suivie d'une ou plusieurs extractions à reflux de ce broyat, ou en soumettant un broyat desdits rhizomes à une percolation à une température comprise entre 10 et 35°C, chacune de ces opérations (macération, extractions à reflux et percolation) étant réalisées au moyen d'un solvant organique ou d'un mélange de solvants organiques appropriés et, si désiré,
* par élimination du ou des solvants de l'extrait ainsi obtenu pour l'obtention d'un extrait sec.

14. Extrait brut selon la revendication 12 ou la revendication 13, **caractérisé en ce qu'**il est un extrait d'*Alpinia galanga*.

15. Extrait purifié d'une plante appartenant au genre *Alpinia*, **caractérisé en ce qu'**il contient une quantité de shogaol selon la revendication 11 au moins égale en poids à 75% du poids sec dudit extrait.

16. Extrait purifié selon la revendication 15, **caractérisé en ce qu'**il est obtenu :
* en soumettant un extrait brut selon l'une quelconque des revendications 12 à 14, après élimination éventuelle du ou des solvants qu'il contient et/ou sa reprise dans l'eau, à une ou plusieurs extractions à contre-courant au moyen d'un solvant organique ou d'un mélange de solvants organiques non miscibles à l'eau et, si désiré,
* par élimination du ou des solvants de l'extrait ainsi obtenu pour l'obtention d'un extrait sec.

17. Extrait purifié selon la revendication 15 ou la revendication 16, **caractérisé en ce qu'**il est un extrait d'*Alpinia galanga*.

18. Composition déodorante, **caractérisée en ce qu'**elle comprend, en tant que substance active, un shogaol selon la revendication 11.

19. Composition déodorante selon la revendication 18, **caractérisée en ce qu'**elle est une composition pharmaceutique qui comprend le [3]-shogaol en tant que principe actif, éventuellement associé à un ou plusieurs autres principes actifs, et au moins un excipient pharmaceutiquement acceptable.

20. Composition déodorante selon la revendication 18, **caractérisée en ce qu'**elle est une composition cosmétique qui comprend le [3]-shogaol en tant que substance active, éventuellement associé à un ou plusieurs autres substances actives, et au moins un excipient convenablement choisi.

21. Composition déodorante selon la revendication 18, **caractérisée en ce qu'**elle est une composition diététique qui comprend le [3]-shogaol en tant que substance active, éventuellement associé à un ou plusieurs autres substances actives, et au moins un excipient convenablement choisi.

## Patentansprüche

1. Verwendung wenigstens einer Verbindung, gewählt aus der Gruppe, die besteht aus den Shogaolen und den Gingerolen zur Herstellung einer deodorierenden Zusammensetzung für den Körper, vorgesehen für eine Verabreichung auf oralem Wege.

2. Verwendung nach Anspruch 1, **dadurch gekennzeichnet, dass** die Verbindung der allgemeinen Formel (l) entspricht: 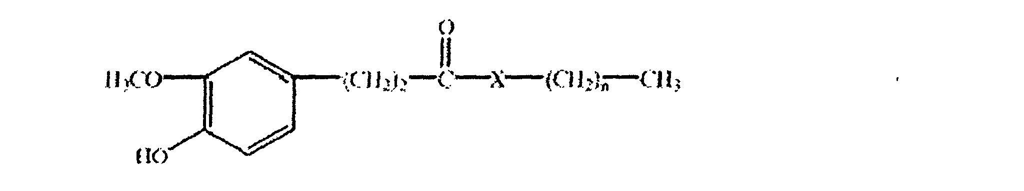 in der X darstellt:
- entweder ein -CH=CH-, in welchem Fall n gleich 1, 2, 4, 6 oder 8 ist;
- entweder -CH₂-CH(OH)-, in dem Fall wo n gleich 2, 4, 6 oder 8 ist.

3. Verwendung nach Anspruch 1 oder Anspruch 2, **dadurch gekennzeichnet, dass** die Verbindung in Form von einem Rohextrakt oder einem gereinigten Extrakt einer Pflanze verwendet wird, die zur Familie der Zingiberaceae gehört, wobei das Extrakt gegebenenfalls einer oder mehreren anderen aktiven Substanzen und/oder einem oder mehreren geeignet gewählten Hilfsstoffen zugeordnet ist.

4. Verwendung nach Anspruch 3, **dadurch gekennzeichnet, dass** das Rohextrakt eine Verbindungsgewichtmenge zwischen 1 und 5 % des Trockengewichts dieses Extrakts enthält.

5. Verwendung nach Anspruch 3 oder Anspruch 4, **dadurch gekennzeichnet, dass** das Rohextrakt der Pflanze, die zur Familie der Zingiberaceae gehört, ausgehend von frischen oder getrockneten Wurzelstöcken der Pflanze erhalten wird :
• durch Mazerierung einer Zermahlung dieser Wurzelstöcke bei einer Temperatur zwischen 10 und 35°C, gefolgt von einer oder mehreren Extraktionen mit Rückfluss von dieser Zermahlung, oder indem man eine Zermahlung dieser Wurzelstöcke einem Perkolieren bei einer Temperatur zwischen 10 und 35°C unterzieht, wobei jeder der Vorgänge (Mazerierung, Extraktionen mit Rückfluss und Perkolieren) mittels eines organischen Lösungsmittels oder eines Gemisches von geeigneten organischen Lösungsmitteln durchgeführt wird, und, wenn gewünscht,
• durch Entfernen des oder der Lösungsmittel von dem so erhaltenen Extrakt zum Erhalt eines Trockenextrakts.

6. Verwendung nach Anspruch 3, **dadurch gekennzeichnet, dass** das gereinigte Extrakt eine Verbindungsmenge enthält, die wenigstens gleich 75 Gew.-% des Trockenextrakts ist.

7. Verwendung nach Anspruch 3 oder Anspruch 6, **dadurch gekennzeichnet, dass** das gereinigte Extrakt der Pflanze, welche zur Familie der Zingiberaceae gehört, erhalten wird:
• indem ein Rohextrakt gemäß Anspruch 4 oder Anspruch 5 nach eventueller Entfernung des oder der Lösungsmittel, die es enthält, und/oder seiner Wiederaufnahme in Wasser einer oder mehreren Extraktionen bei Gegenstrom mittels eines organischen Lösungsmittels oder eines Gemisches von organischen Lösungsmitteln, die nicht mit Wasser mischbar sind, unterzogen wird, und wenn gewünscht,
• durch Entfernen des oder der Lösungsmittel des so erhaltenen Extrakts zum Erhalt eines Trockenextrakts.

8. Verwendung nach einem der Ansprüche 3 bis 7, **dadurch gekennzeichnet, dass** die Pflanze der Familie der Zingiberaceae gewählt wird unter der Gruppe, die umfasst: die Spezies *Alpinia galanga. Alpinia officinarum*, *Zingiber officinalis, Zingiber cassumunar* und *Zingiber zerumbet.*

9. Verwendung nach einem der vorhergehenden Ansprüche, **dadurch gekennzeichnet, dass** die deodorierende Zusammensetzung eine pharmazeutische Zusammensetzung ist, welche die Verbindung als Wirkstoff umfasst, gegebenenfalls zugeordnet zu einem oder mehreren anderen Wirkstoffen und wenigstens einem pharmazeutisch verträglichen Hilfsstoff.

10. Verwendung nach einem der Ansprüche 1 bis 8, **dadurch gekennzeichnet, dass** die deodorierende Zusammensetzung eine Diätzusammensetzung ist, die die Verbindung als aktive Substanz enthält, gegebenenfalls in Verbindung mit einer oder mehren anderen aktiven Substanzen und wenigstens einem geeignet gewählten Hilfsstoff.

11. Shogaol, das der allgemeinen Formel (I) entspricht, in der X -CH=CH- entspricht und n gleich 1 ist.

12. Rohextrakt einer Pflanze, die zur Art *Alpinia* gehört, **dadurch gekennzeichnet, dass** es eine Shogaol-Menge gemäß Anspruch 11 enthält, die an Gewicht zwischen etwa 1 und 5 % des Trockengewichts des Extrakts liegt.

13. Rohextrakt nach Anspruch 12, **dadurch gekennzeichnet, dass** es ausgehend von frischen oder getrockneten Wurzelstöcken der Pflanze erhalten wird:
• durch Mazerierung einer Zermahlung dieser Wurzelstöcke bei einer Temperatur zwischen 10 und 35°C, gefolgt von einer oder mehreren Extraktionen mit Rückfluss von dieser Zermahlung, oder indem man eine Zermahlung dieser Wurzelstöcke einem Perkolieren bei einer Temperatur zwischen 10 und 35°C unterzieht, wobei jeder der Vorgänge (Mazerierung, Extraktionen mit Rückfluss und Perkolieren) mittels eines organischen Lösungsmittels oder eines Gemisches von geeigneten organischen Lösungsmitteln durchgeführt wird, und, wenn gewünscht,
• durch Entfernen des oder der Lösungsmittel von dem so erhaltenen Extrakt zum Erhalt eines Trockenextrakts.

14. Rohextrakt nach Anspruch 12 oder Anspruch 13, **dadurch gekennzeichnet, dass** es ein Extrakt von *Alpinia galanga* ist.

15. Gereinigtes Extrakt einer Pflanze, die zur Art Alpinia gehört, **dadurch gekennzeichnet, dass** es eine Shogaol-Menge nach Anspruch 11 enthält, die wenigstens ab Gewicht gleich 75% des Trockengewichts dieses Extrakts ist.

16. Gereinigtes Extrakt nach Anspruch 15, **dadurch gekennzeichnet, dass** es erhalten wird:
• indem ein Rohextrakt gemäß einem der Ansprüche 12 bis 14 nach eventueller Entfernung des oder der Lösungsmittel, die es enthält, und/oder seiner Wiederaufnahme in Wasser einer oder mehreren Extraktionen bei Gegenstrom mittels eines organischen Lösungsmittels oder eines Gemisches von organischen Lösungsmitteln, die nicht mit Wasser mischbar sind, unterzogen wird, und wenn gewünscht,
• durch Entfernen des oder der Lösungsmittel des so erhaltenen Extrakts zum Erhalt eines Trockenextrakts.

17. Gereinigtes Extrakt nach Anspruch 15 oder Anspruch 16, **dadurch gekennzeichnet, dass** es ein Extrakt von Alpinia galanga ist.

18. Deodorierende Zusammensetzung, **dadurch gekennzeichnet, dass** sie als aktive Substanz ein Shogaol nach Anspruch 11 enthält.

19. Deodorierende Zusammensetzung nach Anspruch 18, **dadurch gekennzeichnet, dass** sie eine pharmazeutische Zusammensetzung ist, die [3]-Shogaol als Wirkstoff umfasst, gegebenenfalls in Verbindung mit einem oder mehreren anderen Wirkstoffen und wenigstens einem pharmazeutisch verträglichen Hilfsstoff.

20. Deodorierende Zusammensetzung nach Anspruch 18, **dadurch gekennzeichnet, dass** sie eine kosmetische Zusammensetzung ist, die [3]-Shogaol als aktive Substanz umfasst, gegebenenfalls in Verbindung mit einer oder mehreren aktiven Substanzen und wenigstens einem geeignet gewählten Hilfsstoff.

21. Deodorierende Zusammensetzung nach Anspruch 18, **dadurch gekennzeichnet, dass** sie eine diätetische Zusammensetzung ist, die [3]-Shogaol als aktive Substanz umfasst, gegebenenfalls in Verbindung mit einer oder mehreren anderen aktiven Substanzen und wenigstens einem geeignet gewählten Hilfsstoff.

## Claims

1. Use of at least one component chosen from the group of shogaols and gingerols for the preparation of a body deodorising compound intended for administration via the oral route.

2. Use according to Claim 1, **characterised by** the fact that the component satisfies the general formula (I): 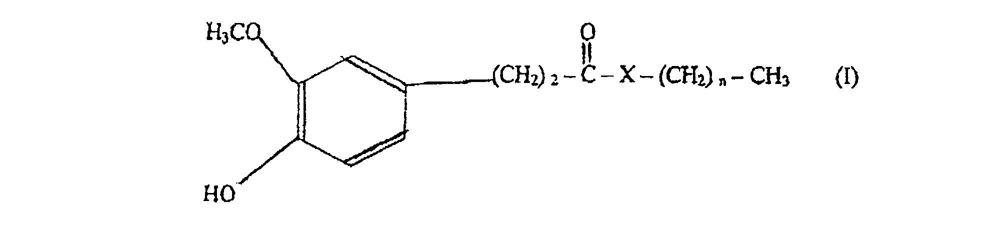 in which x represents:
- either -CH=CH-, in which case n is equal to 1, 2, 4, 6 or 8;
- or -CH₂-CH(OH)-, in which case n is equal to 2, 4, 6 or 8.

3. Use according to Claim 1 or Claim 2, **characterised by** the fact that the component is used in the form of an unrefined or purified extract from a plant belonging to the family of Zingiberaceae, this extract possibly being combined with one or more other active substances and/or one or more of the appropriately chosen excipients.

4. Use according to Claim 3, **characterised by** the fact that this unrefined extract contains a quantity of component whose weight is between 1% and 5% of the dry weight of this extract.

5. Use according to to Claim 3 or Claim 4, **characterised by** the fact that the unrefined extract from the plant belonging to the family of Zingiberaceae is obtained from fresh or dry rhizomes of the said plant:
∗ by maceration of a homogenate of these rhizomes at a temperature of between 10°C and 35°C followed by one or more extractions under reflux of this homogenate or by subjecting a homogenate of the said rhizomes to percolation at a temperature of between 10°C and 35°C, with each of these operations (maceration, extraction under reflux and percolation) being performed using an organic solvent or a mixture of suitable organic solvents and, if desired,
∗ by elimination of the solvent(s) from the extract obtained thus, for the purposes of obtaining a dry extract.

6. Use according to Claim 3, **characterised by** the fact that the purified extract contains a quantity of component which is at least equal in weight to 75% of the dry weight of this extract.

7. Use according to Claim 3 or Claim 6, **characterised by** the fact that the purified extract of the plant belonging to the family of Zingiberaceae is obtained:
∗ by subjecting an unrefined extract according to Claim 4 or Claim 5, after the possible elimination of the solvent(s) which it contains and/or its recovery in water, 10 one or more countercurrent extractions with the use of an organic solvent or a mixture of organic solvents which are immiscible with water and, if desired,
∗ by elimination of the solvent(s) from the extract obtained thus, for the purposes of obtaining a dry extract.

8. Use according to any of claims 3 to 7, **characterised by** the fact that the plant of the family of Zingiberaceae is chosen from the group which includes the species *Alpinia galanga, Alpinia officinarum, Zingiber officinalis, Zingiber cassumunar* and *Zingiber zerumbet*.

9. Use according to any of the previous claims, **characterised by** the fact that the deodorising compound is a pharmaceutical compound which contains the component as the active principle, possibly combined with one or more other active principles and at least one pharmaceutically acceptable excipient.

10. Use according to any of claims 1 to 8, **characterised by** the fact that the deodorising compound is a dietetic compound which contains the component as the active substance, possibly combined with one or more other active substances and at least one appropriately chosen excipient.

11. Shogaol which satisfies the general formula (I) in which x represents -CH=CH- and n is equal to 1.

12. Unrefined extract from a plant belonging to the genus *Alpinia*, **characterised by** the fact that it contains a quantity of shogaol according to Claim 11 whose weight is between about 1 and 5% of the dry weight of the said extract.

13. Unrefined extract according to Claim 12, **characterised by** the fact that it is obtained from fresh or dry rhizomes of the said plant:
∗ by maceration of a homogenate of the said rhizomes at a temperature of between 10°C and 35°C followed by one or more extractions under reflux of this homogenate or by subjecting a homogenate of the said rhizomes to percolation at a temperature of between 10°C and 35°C, with each of these operations (maceration, extraction under reflux and percolation) being performed using an organic solvent or a mixture of suitable organic solvents and, if desired,
∗ by elimination of the solvent(s) from the extract obtained thus, for the purposes of obtaining a dry extract.

14. Unrefined extract according to Claim 12 or Claim 13, **Characterised by** the fact that it is an extract of *Alpinia galanga*.

15. Purified extract from a plant belonging to the genus *Alpinia*, **characterised by** the fact that it contains a quantity of shogaol according to Claim 11 which is at least equal in weight to 75% of the dry weight of the said extract.

16. Purified extract according to Claim 15, **characterised by** the fact that it is obtained:
∗ after the possible elimination of the solvent(s) which it contains and/or its recovery in water, by subjecting an unrefined extract according to any of claims 12 to 14 to one or more countercurrent extractions using an organic solvent or a mixture of organic solvents which are immiscible with water and, if desired,
∗ by elimination of the solvent(s) from the extract obtained thus, for the purposes of obtaining a dry extract.

17. Purified extract according to Claim 15 or Claim 16, **characterised by** the fact that it is an extract of *Alpinia galanga*.

18. Deodorising compound, **characterised by** the fact that it contains a shogoal according to Claim 11 as the active substance.

19. Deodorising compound according to Claim 18, **characterised by** the fact that it is a pharmaceutical compound which includes [3]-shogaol as the active principle, possibly combined with one or more other active principles and at least one pharmaceutically acceptable excipient.

20. Deodorising compound according to Claim 18, **characterised by** the fact that it is a cosmetic compound which includes [3]-shogaol as the active substance, possibly combined with one or more other active substances and at least one appropriately chosen excipient.

21. Deodorising compound according to Claim 18, **characterised by** the fact that it is a dietetic compound which includes [3]-shogaol as the active substance, possibly combined with one or more other active substances and at least one appropriately chosen excipient.
